# EUROPÄISCHE PATENTANMELDUNG

(11) **EP 1 634 623 A2**
(43) Veröffentlichungstag der Anmeldung: **15.03.2006**
(21) Anmeldenummer: 05107522.4
(22) Anmeldetag: 16.08.2005
(51) Int. Cl.: A61Q 17/04, A61K 8/49

(54) **Fließfähige kosmetische Zubereitung mit Lichtschutzfiltern**

(30) Priorität: 16.08.2004 DE 102004039726
(71) Anmelder: Beiersdorf Aktiengesellschaft, 20253 Hamburg (DE)
(72) Erfinder: Mundt, Claudia, 28207, Bremen (DE); Müller, Anja Sabine, 26789, Leer (DE); Tesch, Mirko, 22303, Hamburg (DE)

(57) **Zusammenfassung**

Kosmetische Zubereitung enthaltend
a) eine UV-Lichtschutzfilterkombination aus
   i) 4,4',4"-(1,3,5-Triazin-2,4,6-triyltriimino)-tris-benzoësäure-tris(2-ethylhexylester) und/oder Dioctylbutylamidotriazon sowie
   ii) 2,4-bis-[5-1(dimethylpropyl)benzoxazol-2-yl-(4-phenyl)-imino]-6-(2-ethylhexyl)-imino-1,3,5-triazin und
b) eine oder mehrere Ölkomponenten mit mindestens einer Hydroxyl-Gruppe, die dadurch gekennzeichnet ist, dass der oder die Ölkomponenten mit mindestens einer Hydroxyl-Gruppe zu mindestens 10 Gewichts-%, bezogen auf das Gesamtgewicht der Ölphase, in der Zubereitung enthalten sind.

## Beschreibung

Die vorliegende Erfindung betrifft eine kosmetische Zubereitung enthaltend eine UV-Lichtschutzfilterkombination aus 4,4',4"-(1,3,5-Triazin-2,4,6-triyltriimino)-tris-benzoësäure-tris(2-ethylhexylester) und/oder Dioctylbutylamidotriazon sowie 2,4-bis-[5-1(dimethylpropyl)benzoxazol-2-yl-(4-phenyl)-imino]-6-(2-ethylhexyl)-imino-1,3,5-triazin und eine oder mehrere Ölkomponenten mit mindestens einer Hydroxyl-Gruppe.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB-und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurden daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie dem Anlage 7 der Kosmetikverordnung zusammengefasst.

Eine besonders wirkungsvolle Klasse von in der Kosmetik verwendbaren UV-Lichtschutzfiltersubstanzen stellen Verbindungen dar, die ein Triazin-Grundgerüst aufweisen.

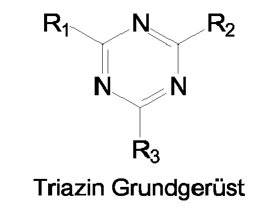

Hierzu zählen beispielsweise die Lichtschutzfilter
- 2,4-Bis-{[4-(2-ethyl-hexyloxy)-2-hydroxy]-phenyl}-6-(4-methoxyphenyl)-1,3,5-triazin (INCI: Aniso Triazin, erhältlich unter dem Handelsnamen Tinosorb S),
- Dioctylbutylamidotriazon (INCI: Dioctylbutamidotriazone),
- 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0, erhältlich bei 3V Sigma unter der Handelsbezeichnung Uvasorb® K2A),
- 4,4',4"-(1,3,5-Triazin-2,4,6-triyltriimino)-tris-benzoësäure-tris(2-ethylhexylester) (auch: 2,4,6-Tris-[anilino-(p-carbo-2'-ethyl-1'-hexyloxy)]-1,3,5-triazin (INCI: Octyl Triazone), welches von der BASF Aktiengesellschaft unter der Warenbezeichnung UVINUL® T 150 vertrieben wird).

Insbesondere die Verbindungen 4,4',4"-(1,3,5-Triazin-2,4,6-triyltriimino)-tris-benzoësäure-tris(2-ethylhexylester) (UVINUL® T 150) und 2,4-bis-[5-1 (dimethylpropyl)benzoxazol-2-yl-(4-phenyl)-imino]-6-(2-ethylhexyl)-imino-1,3,5-triazin (Uvasorb® K2A) haben jedoch den Nachteil, dass sie nur schwierig in kosmetische Zubereitungen einzuarbeiten sind. Dies liegt vor allem an ihrer geringen Löslichkeit in der Ölphase kosmetischer Zubereitungen sowie an ihrer Neigung in den Zubereitungen zu kristallisieren und wieder auszufallen.

Es hat daher in der Vergangenheit nicht an Versuchen gefehlt, Ölkomponenten zu finden bzw. Rezepturen zu entwickeln, in denen sich größere Mengen an diesen UV-Filtersubstanzen stabil einarbeiten lassen. So gibt es eine Reihe von Ölen, die symetrische und/oder unsymetrisch substituierte Triazine lösen können. Zu diesen Ölen gehört beispielsweise das Diethylhexyl 2,6-Naphthalate (DE 10008894), das Dibutyladipat (DE 10038713), das Butylenglycol Dicaprylat/Dicaprat (DE 19703471), das Dicicaprylylcarbonat (DE 10038712) oder Octyldodecanol (DE 19651057). Zudem sind weitere Öle bekannt die ein gutes Lösevermögen für UV-Filter besitzen wie das C12-15 Alkylbenzoat.

Nachteilig am Stande der Technik war jedoch der Umstand, dass sich zwar die einzelnen erfindungsgemäßen UV-Filter in diesen Ölen lösen lassen. Bei Zusammengabe der getrennt gelösten UV-Filter kommt es jedoch zu Auskristallisation von UV-Filtern. Daher konnten bereits bekannte Erkenntnisse zum Löslichkeitsverhalten von UV-Filtern nicht den Weg zum Lösen der vorliegenden Erfindung weisen.

Nachteilig am Stande der Technik ist ferner der Umstand, dass es bei der Weiterverarbeitung der vereinigten Ölphasen zu kosmetischen Zubereitungen in Emulsionsform im Verlaufe der Zeit zu einem Anstieg der Viskosität und zu einer Verfestigung der Zubereitung kommt. Die Emulsion ist spätestens nach einigen Tagen nicht mehr fließfähig sondern vielmehr schnittfest und lässt sich nicht mehr auf die Haut applizieren.

Als weiteren Stand der Technik kennt der Fachmann die DE 101 62 841, die Zubereitungen enthaltend Ölkomponenten mit einer geringen Oberflächenspannung und Benzoxazolderivaten wie beispielsweise 2,4-bis-[5-1(dimethylpropyl)benzoxazol-2-yl-(4-phenyl)-imino]-6-(2-ethylhexyl)-imino-1,3,5-triazin offenbart.

Ferner beschreibt die DE 196 15 038 Zubereitungen enthaltend 4,4',4"-(1,3,5-Triazin-2,4,6-triyltriimino)-tris-benzoësäure-tris(2-ethylhexylester) und α, β-Dihydroxydicarbonsäuren.

Es war die Aufgabe der vorliegenden Erfindung, die Mängel des Standes der Technik zu beseitigen und langzeitstabile, fließfähige Zubereitungen enthaltend eine Kombination aus 2,4-bis-[5-1 (dimethylpropyl)benzoxazol-2-yl-(4-phenyl)-imino]-6-(2-ethylhexyl)-imino-1,3,5-triazin und mindestens einem weiteren UV-Lichtschutzfilter aus der Gruppe der Triazinderivate zu entwickeln.

Überraschend gelöst werden die Aufgaben durch eine kosmetische Zubereitung enthaltend
a) eine UV-Lichtschutzfilterkombination aus
   i) 4,4',4"-(1,3,5-Triazin-2,4,6-triyltriimino)-tris-benzoësäure-tris(2-ethylhexylester) und/oder Dioctylbutylamidotriazon sowie
   ii) 2,4-bis-[5-1(dimethylpropyl)benzoxazol-2-yl-(4-phenyl)-imino]-6-(2-ethylhexyl)-imino-1,3,5-triazin und
b) eine oder mehrere Ölkomponenten mit mindestens einer Hydroxyl-Gruppe, die dadurch gekennzeichnet ist, dass der oder die Ölkomponenten mit mindestens einer Hydroxyl-Gruppe zu mindestens 10 Gewichts-%, bezogen auf das Gesamtgewicht der Ölphase, in der Zubereitung enthalten sind.

Ferner werden die Aufgaben gelöst durch die Verwendung von Ölkomponenten mit mindestens einer Hydroxyl-Gruppe zur Reduzierung der Viskosität von kosmetischen Zubereitungen welche eine UV-Lichtschutzfilterkombination aus
i) 4,4',4"-(1,3,5-Triazin-2,4,6-triyltriimino)-tris-benzoësäure-tris(2-ethylhexylester) und/oder Dioctylbutylamidotriazon sowie
ii) 2,4-bis-[5-1(dimethylpropyl)benzoxazol-2-yl-(4-phenyl)-imino]-6-(2-ethylhexyl)-imino-1,3,5-triazin, enthalten.

Unter erfindungsgemäßen Zubereitungen werden im Rahmen dieser Schrift die erfindungsgemäßen kosmetischen Zubereitungen verstanden und Zubereitungen in denen die erfindungsgemäße Verwendung verwirklicht ist.

Zwar werden im Stand der Technik Zubereitungen beschrieben, welche mehrere Triazinderivate enthalten, beispielsweise EP 1 027 883 und EP 1 300 137. Diese Schriften konnten jedoch nicht den Weg zur vorliegenden Erfindung weisen.

Erfindungsgemäßen kosmetischen Zubereitungen können in verschiedenen Formen vorliegen. So können sie z.B. eine wasserfreie Zubereitung, eine Emulsion oder Mikroemulsion vom Typ Wasser-in-Öl (W/O) oder vom Typ Öl-in-Wasser (O/W), eine multiple Emulsionen, beispielsweise vom Typ Wasser-in-Öl-in-Wasser (W/O/W), einen festen Stift, eine Salbe, einen Schaum oder auch ein Aerosol darstellen.

Erfindungsgemäß sind die kosmetischen Zubereitungen ferner, wenn sie in Form einer Mehrfachemulsion, Mikroemulsion, Pickering-Emulsion oder sprühbarer Emulsion und ähnlichem vorliegen.

Erfindungsgemäß bevorzugte Ausführungsformen der vorliegenden Erfindung sind dadurch gekennzeichnet, dass für die kosmetischen Zubereitungen oder Verwendungen die Ölkomponenten mit mindestens einer Hydroxyl-Gruppe gewählt werden aus der Gruppe organischen Hydroxyester. Erfindungsgemäß besonders bevorzugt ist es dabei, wenn die Ölkomponenten mindestens zwei Hydroxylgruppe besitzen. In diesem Falle ist es erfindungsgemäß vorteilhaft, wenn die Ölkomponente mit mindestens einer Hydroxyl-Gruppe ein Di- oder Tricarbonsäureester gewählt wird. Erfindungsgemäß vorteilhafte Öle sind beispielsweise Tartrate, Lactate, Citrate, Malate und Ricinusöl. Erfindungsgemäß besonders bevorzugt sind Tartrate insbesondere das Di- C12-13 Alkyltatrat (Cosmacol ETI).

Erfindungsgemäß besonders bevorzugt ist es, als Ölkomponente ein Dihydroxydicarbonsäureester einzusetzen.

Erfindungsgemäß am stärksten bevorzugt sind erfindungsgemäße kosmetische Zubereitungen, Herstellungsverfahren und/oder Verwendungen, die dadurch gekennzeichnet sind, dass als Ölkomponente das Di-C12-13 Alkyltartrat eingesetzt wird.

Erfindungsgemäß bevorzugte Ausführungsformen der vorliegenden Erfindung sind ferner dadurch gekennzeichnet, dass eine UV-Lichtschutzfilterkombination aus 4,4',4"-(1,3,5-Triazin-2,4,6-triyltriimino)-tris-benzoësäure-tris(2-ethylhexylester) und 2,4-bis-[5-1(dimethylpropyl)benzoxazol-2-yl-(4-phenyl)-imino]-6-(2-ethylhexyl)-imino-1,3,5-triazin eingesetzt wird.

Auch ist es vorteilhaft im Sinne der vorliegenden Erfindung, wenn die UV-Lichtschutzfilterkombination in einer Gesamtmenge von 0,1 bis 10 Gewichts-%, und bevorzugt in einer Gesamtmenge von 0,5 bis 8 Gewichts-%, jeweils bezogen auf das Gesamtgewicht der Zubereitung, in der Zubereitung enthalten ist.

In den erfindungsgemäß bevorzugten Ausführungsformen der vorliegenden Erfindung beträgt das Gewichtsverhältnis von 4,4',4"-(1,3,5-Triazin-2,4,6-triyltriimino)-tris-benzoësäure-tris(2-ethylhexylester) zu 2,4-bis-[5-1(dimethylpropyl)benzoxazol-2-yl-(4-phenyl)-imino]-6-(2-ethylhexyl)-imino-1,3,5-triazin von 0,1 : 8 bis 8 : 0,1 und besonders bevorzugt von 0,5 : 6 bis 6 : 0,5.

Erfindungsgemäß vorteilhafte Ausführungsformen der vorliegenden Erfindung sind ferner dadurch gekennzeichnet, dass das Gewichtsverhältnis von UV-Lichtschutzfilterkombination zur Gesamtmenge an Ölkomponenten mit mindestens einer Hydroxyl-Gruppe von 2 : 1 bis 1 : 20 und bevorzugt von 1, 5 : 1 bis 1 : 15 beträgt.

Das erfindungsgemäße 2,4-bis-[5-1(dimethylpropyl)benzoxazol-2-yl-(4-phenyl)-imino]-6-(2-ethylhexyl)-imino-1,3,5-triazin mit der CAS Nr. 288254-16-0 ist u.a. erhältlich bei 3V Sigma unter der Handelsbezeichnung Uvasorb® K2A.

Das erfindungsgemäße 4,4',4"-(1,3,5-Triazin-2,4,6-triyltriimino)-tris-benzoësäure-tris(2-ethyl-hexylester) (auch: 2,4,6-Tris-[anilino-(p-carbo-2'-ethyl-1'-hexyloxy)]-1,3,5-triazin (INCI: Octyl Triazone), wird beispielsweise von der BASF Aktiengesellschaft unter der Warenbezeichnung UVINUL® T 150 vertrieben.

Das erfindungsgemäße Dioctylbutylamidotriazon (INCI: Diethylhexyl Butamido Triazone) ist unter der Handelsbezeichnung UVASORB HEB bei Sigma 3V erhältlich.

Die erfindungsgemäß vorteilhaften Ausführungsformen der vorliegenden Erfindung sind dadurch gekennzeichnet, dass die Viskosität der Zubereitung weniger als 30000 mPas und besonders bevorzugt weniger als 12000 mPas beträgt.
Insbesondere sollte der Viskositätsanstieg während der Lagerung weniger als 10000 mPa insbesondere weniger als 5000mPa betragen.

Die im Rahmen der vorliegenden Schrift aufgeführten Viskositätswerte der Zubereitungen und Einzelsubstanzen wurden mit Hilfe eines Viskosimeters des Typs Viskotester VT 02 der Gesellschaft Haake ermittelt (Temperatur (falls nicht anders angegegben): 20°C, Spindeldurchmesser 24 mm, Rotorgeschwindigkeit 62 min⁻¹).

Die erfindungsgemäßen Zubereitungen können darüber hinaus weitere UV-Lichtschutzfiltersubstanzen enthalten.

Enthalten die erfindungsgemäßen Zubereitungen UVB-Filtersubstanzen, können diese öllöslich oder wasserlöslich sein.

Erfindungsgemäß vorteilhafte öllösliche UVB-Filter sind z.B.:
- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester, 2-(4'-Diethylamino-2'-hydoxybenzoyl)-benzoesäurehexylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure(4-isopropylbenzyl)ester, Salicylsäurehomomenthylester,
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester,
- Ester der 2-Cyano-3,3-Diphenylacrylsäure, bevorzugt Ethylhexyl-2-cyano-3,3-diphenyl-acrylat,
- Diethylhexyl-butamidotriazon, 2,4,6-Trianilino-(p-carbo-2'-ethyl-1'-hexyloxy) -1,3,5-triazin
- 3-(4-(2,2-bis Ethoxycarbonylvinyl)-phenoxy)propenyl)-methoxysiloxan / Dimethylsiloxan
- Copolymer,
- 2-(2H-benzotriazol-2-yl)-4-methyl-6-[2-methyl-3- [1,3,3,3 -tetramethyl-1-[(trimethylsilyl)oxy]disiloxanyl]propyl]-phenol (CAS-Nr.: 155633-54-8).

Vorteilhafte wasserlösliche UVB-Filter sind z.B.:
- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und ihre Salze sowie das 1,4-di(2-oxo-10-Sulfo-3-bornylidenmethyl)-Benzol und dessen Salze (die entprehenden 10-Sulfato-verbindungen, beispielsweise das entsprechende Natrium-, Kalium- oder Triethanolammonium-Salz), auch als Benzol-1,4-di(2-oxo-3-bornylidenmethyl-10-Sulfonsäure bezeichnet.

Die Liste der genannten UVB-Filter, die in Kombination mit den erfindungsgemäßen Wirkstoffkombinationen verwendet werden können, soll selbstverständlich nicht limitierend sein.

Gegenstand der Erfindung ist auch die Verwendung einer Kombination der erfindungsgemäß verwendeten Emulsionen mit mindestens einem UVB-Filter als Antioxidans bzw. die Verwendung einer Kombination der erfindungsgemäß verwendeten Wirkstoffkombinationen mit mindestens einem UVB-Filter als Antioxidans in einer kosmetischen oder dermatologischen Zubereitung.

Es kann auch von Vorteil sein, UVA-Filter einzusetzen, die üblicherweise in kosmetischen Zubereitungen enthalten sind. Bei diesen Substanzen handelt es sich vorzugsweise um Derivate des Dibenzoylmethans, insbesondere um 1-(4'-tert.Butylphenyl)-3-(4'-methoxyphenyl)-propan-1,3-dion und um 1-Phenyl-3-(4'-isopropylphenyl)propan-1,3-dion.

Weiterhin vorteilhafte UVA-Filter aus der Gruppe der Triazine sind z.B. das 2,4-Bis-{[4-(2-Ethyl-hexyloxy)-2-hydroxy]-phenyl}-6-(4-methoxyphenyl)-1,3,5-triazin (Handelsbezeichnung Tinosorb® S) sowie das 2,2'-Methylen-bis- [6-2H-benzotriazol-2yl]-4-(1,1,3,3-tetramethylbutyl)phenol) (Handels-bezeichnung Tinosorb® M). Ein vorteilhafter wasserlöslicher UVA-Filter stellt das 2'-bis-(1,4-Phenylen)-1H-benzimidazol-4,6-disulfonsäure-Natriumsalz dar (Handelsbezeichnung Neo Heliopan AP®).

Es können die für die UVB-Kombination verwendeten Mengen eingesetzt werden.

Bevorzugte anorganische Pigmente sind Metalloxide und/oder andere in Wasser schwerlösliche oder unlösliche Metallverbindungen, insbesondere Oxide des Titans (TiO₂), Zinks (ZnO), Eisens (z. B. Fe₂O₃), Zirkoniums (ZrO₂), Siliciums (SiO₂), Mangans (z. B. MnO), Aluminiums (Al₂O₃), Cers (z. B. Ce₂O₃), Mischoxide der entsprechenden Metalle sowie Abmischungen aus solchen Oxiden sowie das Sulfat des Bariums (BaSO₄).

Die Pigmente können vorteilhaft im Sinne der vorliegenden Erfindung auch in Form kommerziell erhältlicher öliger oder wässriger Vordispersionen zur Anwendung kommen. Diesen Vordispersionen können vorteilhaft Dispergierhilfsmittel und/oder Solubilisationsvermittler zugesetzt sein.

Die Pigmente können erfindungsgemäß vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, dass die Pigmente nach an sich bekannten Verfahren mit einer dünnen hydrophilen und/oder hydrophoben anorganischen und/oder organischen Schicht versehen werden. Die verschiedenen Oberflächenbeschichtungen können im Sinne der vorliegenden Erfindung auch Wasser enthalten.

Anorganische Oberflächenbeschichtungen im Sinne der vorliegenden Erfindung können bestehen aus Aluminiumoxid (Al₂O₃), Aluminiumhydroxid Al(OH)₃, bzw. Aluminiumoxidhydrat (auch: Alumina, CAS-Nr.: 1333-84-2), Natriumhexametaphosphat (NaPO₃)₆, Natriummetaphosphat (NaPO₃)ₙ, Siliciumdioxid (SiO₂) (auch: Silica, CAS-Nr.: 7631-86-9), oder Eisenoxid (Fe₂O₃). Diese anorganischen Oberflächenbeschichtungen können allein, in Kombination und/oder in Kombination mit organischen Beschichtungsmaterialien vorkommen.

Organische Oberflächenbeschichtungen im Sinne der vorliegenden Erfindung können bestehen aus pflanzlichem oder tierischem Aluminiumstearat, pflanzlicher oder tierischer Stearinsäure, Laurinsäure, Dimethylpolysiloxan (auch: Dimethicone), Methylpolysiloxan (Methicone), Simethicone (einem Gemisch aus Dimethylpolysiloxan mit einer durchschnittlichen Kettenlänge von 200 bis 350 Dimethylsiloxan-Einheiten und Silicagel) oder Alginsäure. Diese organischen Oberflächenbeschichtungen können allein, in Kombination und/oder in Kombination mit anorganischen Beschichtungsmaterialien vorkommen.

Erfindungsgemäß geeignete Zinkoxidpartikel und Vordispersionen von Zinkoxidpartikeln sind unter folgenden Handelsbezeichnungen bei den aufgeführten Firmen erhältlich:

| | | |
|---|---|---|
| Handelsname | Coating | Hersteller |
| Z- Cote HP1 | 2% Dimethicone | BASF |
| Z- Cote | / | BASF |
| ZnO NDM | 5% Dimethicone | H&R |

Geeignete Titandioxidpartikel und Vordispersionen von Titandioxidpartikeln sind unter folgenden Handelsbezeichnungen bei den aufgeführten Firmen erhältlich:

| | | |
|---|---|---|
| Handelsname | Coating | Hersteller |
| MT-100TV | Aluminiumhydroxid / Stearinsäure | Tayca Corporation |
| MT-100Z | Aluminiumhydroxid / Stearinsäure | Tayca Corporation |
| Eusolex T-2000 | Alumina / Simethicone | Merck KgaA |
| Titandioxid T805 (Uvinul TiO₂) | Octyltrimethylsilan | Degussa |

Die Ölphase der erfindungsgemäßen Zubereitung kann vorteilhaft weitere Ölkomponenten enthalten. Diese können vorteilhaft gewählt werden aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.

Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs).

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Octylpalmitat, Octylcocoat, Octylisostearat,

Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann die Ölphase vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung *Cetiol CC* bei der Fa. Cognis erhältliche.

Es ist ferner bevorzugt, das oder die Ölkomponenten aus der Gruppe Isoeikosan, Neopentylglykoldiheptanoat, Propylenglykoldicaprylat/dicaprat, Caprylic/Capric/Diglycerylsuccinat, Butylenglykol Dicaprylat/Dicaprat, Triisostearin, Dipentaerythrityl Hexacaprylat/Hexacaprat, Propylenglykolmonoisostearat, Tricaprylin, Dimethylisosorbid.

Vorteilhafte Ölkomponenten sind ferner z. B. Butyloctylsalicylat (beispielsweise das unter der Handelsbezeichnung *Hallbrite BHB* bei der Fa. CP Hall erhältliche), Hexadecylbenzoat und Butyloctylbenzoat und Gemische davon (*Hallstar AB*) und/oder Diethylhexylnaphthalat (*Hallbrite TQ oder Corapan TQ von H&R*).

Auch beliebige Abmischungen solcher Öl- und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene und Isohexadecan. Unter den Polyolefinen sind Polydecene die bevorzugten Substanzen.

Vorteilhaft kann die Ölphase ferner einen Gehalt an zyklischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Silikonöle sind hochmolekulare synthetische polymere Verbindungen, in denen SiliciumAtome über Sauerstoff-Atome ketten- und/oder netzartig verknüpft und die restlichen Valenzen des Siliciums durch Kohlenwasserstoff-Reste (meist Methyl-, seltener Ethyl-, Propyl-, Phenyl-Gruppen u. a.) abgesättigt sind. Systematisch werden die Silikonöle als Polyorganosiloxane bezeichnet. Die methylsubstituierten Polyorganosiloxane, welche die mengenmäßig bedeutendsten Verbindungen dieser Gruppe darstellen und sich durch die folgende Strukturformel auszeichnen 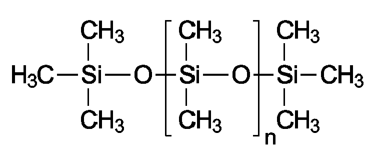 werden auch als Polydimethylsiloxan bzw. Dimethicon (INCI) bezeichnet. Dimethicone gibt es in verschiedenen Kettenlängen bzw. mit verschiedenen Molekulargewichten.

Besonders vorteilhafte Polyorganosiloxane im Sinne der vorliegenden Erfindung sind beispielsweise Dimethylpolysiloxane [Poly(dimethylsiloxan)], welche beispielsweise unter den Handelsbezeichnungen Abil 10 bis 10000 bei Th. Goldschmidt erhältlich sind. Ferner vorteilhaft sind Phenylmethylpolysiloxane (INCI: Phenyl Dimethicone, Phenyl Trimethicone), zyklische Silikone (Octamethylcyclotetrasiloxan bzw. Decamethylcyclopentasiloxan), welche nach INCI auch als Cyclomethicone bezeichnet werden, aminomodifizierte Silikone (INCI: Amodimethicone) und Silikonwachse, z. B. Polysiloxan-Polyalkylen-Copolymere (INCI: Stearyl Dimethicone und Cetyl Dimethicone) und Dialkoxydimethylpolysiloxane (Stearoxy Dimethicone und Behenoxy Stearyl Dimethicone), welche als verschiedene Abil-Wax-Typen bei Th. Goldschmidt erhältlich sind. Aber auch andere Silikonöle sind vorteilhaft im Sinne der vorliegenden Erfindung zu verwenden, beispielsweise Cetyldimethicon, Hexamethylcyclotrisiloxan, Polydimethylsiloxan, Poly(methylphenylsiloxan).

Liegt die erfindungsgemäße Zubereitung in Form einer O/W-Emulsion vor, so lässt sich diese grundsätzlich mit allen bekannten O/W-Emulgatoren und Emulgatorkombinationen herstellen.

Erfindungsgemäß vorteilhaft ist es einen O/W-Emulgator oder eine O/W-Emulgatorkombination gewählt aus den folgenden O/W-Emulgatoren:
i) Glycerylstearatcitrat,
ii) Cetearylalkohol + PEG-40 hydriertes Rizinusöl + Natriumcetearylsulfat + Glycerylstearat,
iii) Polyglyceryl-3-methylglucosedistearat,
iv) Stearinsäure
v) PEG-40 Stearat
vi) PEG-100 Stearat oder
vii) Kaliumcetylphosphat

einzusetzen.

Dabei ist es erfindungsgemäß bevorzugt, die O/W-Emulgatoren aus der Liste der folgenden Verbindungen zu wählen:
i) Glycerylstearatcitrat,
ii) Cetearylalkohol + PEG-40 hydriertes Rizinusöl + Natriumcetearylsulfat + Glycerylstearat,
iii) Polyglyceryl-3-methylglucosedistearat oder
iv) Stearinsäure
v) PEG-40 Stearat.

Erfindungsgemäß besonders bevorzugt ist der Einsatz der O/W-Emulgatoren Glycerylstearatcitrate+ PEG-40 hydriertes Rizinusöl + Natriumcetearylsulfat + Glycerylstearat, Polyglyceryl-3-methylglucosedistearat.

Als erfindungsgemäß vorteilhafte W/O-Emulgatoren können oberflächenaktive Verbindungen eingesetzt werden, die einen HLB-Wert von 3 bis 8 aufweisen.

Erfindungsgemäß bevorzugt werden dabei ein oder mehrere W/O-Emulgatoren gewählt aus der Gruppe der folgenden Verbindungen eingesetzt:
Sorbitanstearat, Sorbitanoleat, Lecithin, Glyceryllanolat, Lanolin, Ozokerit, hydriertes Ricinusöl, Glycerylisostearat, Polyglyceryl-3-Oleat, Wollwachssäuregemische, Wollwachsalkoholgemische, Pentaerythrithylisostearat, Polyglyceryl-3-diisostearat, Methylglucosedioleat, PEG-7-hydriertes Ricinusöl, Polyglyceryl-4-isostearat, Hexyllaurat, Acrylat/ C₁₀₋₃₀-Alkylacrylat-Crosspolymer, Sorbitanisostearat, Poloxamer 101, Polyglyceryl-2-dipolyhydroxystearat, Polyglyceryl-4-dipolyhydroxystearat, PEG-30-dipolyhydroxystearat, Diisostearoylpolyglyceryl-3-diisostearat, Polyglyceryl-3-dipolyhydroxystearat, Polyglyceryl-3-dioleat.

Dabei sind unter anderem die folgenden Kombinationen aus lipophilen Bestandteilen und Emulgatoren erfindungsgemäß vorteilhaft:
Sorbitanoleat im Gemisch mit hydriertem Ricinusöl, Bienenwachs (Cera alba) und Stearinsäure, Natriumdihydroxycetylphosphat im Gemisch mit Isopropylhydroxycetylether, Methylglucosedioleat im Gemisch mit Hydroxystearat und Bienenwachs, Mineralöl im Gemisch mit Petrolatum, Ozokerit, Glyceryloleat und Lanolinalkohol, Petrolatum im Gemisch mit Ozokerit, hydriertem Ricinusöl, Glycerylisostearat und Polyglyceryl-3-oleat, Sorbitanoleat im Gemisch mit PEG-2-hydriertem Ricinusöl, Ozokerit und hydriertem Ricinusöl, Sorbitanisostearat im Gemisch mit PEG-2-hydriertem Ricinusöl.

Es ist erfindungsgemäß besonders bevorzugt, wenn als W/O-Emulgatoren Polyglyceryl-2 Dipolyhydroxystearat oder PEG-30 Dipolyhydroxystearat eingesetzt werden.

Vorteilhafte Ausführungsformen der vorliegenden Erfindung sind dadurch gekennzeichnet, dass sie Emulgatoren in einer Gesamtkonzentration von 0,1 bis 10 Gewichts-%, bezogen auf das Gesamtgewicht der Zubereitung, enthalten. Erfindungsgemäß bevorzugt ist es dabei, wenn die Gesamtkonzentration an Emulgatoren von 0,5 bis 5 Gewichts-%, bezogen auf das Gesamtgewicht der Zubereitung beträgt.
Unter den Emulsionen stellen die O/W_Emulsionen die erfindungsgemäß bevorzugte Ausführungsform dar.

Die Wasserphase einer erfindungsgemäßen Emulsion kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Butylenglykol, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, Polymere, Schaumstabilisatoren, Elektrolyte sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole [von der Fa. Bf. Goodrich], beispielsweise Carbopole der Typen 980, 981, 1382, 2984, 5984, ETD 2020, ETD 2050, Ultrez 10, jeweils einzeln oder in Kombination.

Die erfindungsgemäßen Zubereitungen können ferner vorteilhaft auch Selbstbräunungssubstanzen enthalten, wie beispielsweise Dihydroxyacteon und/oder Melaninderivate in Konzentrationen von 1 Gew.-% bis zu 10 Gew.-%, bezogen auf das Gesamtgewicht der Zubereitung.

Ferner vorteilhaft können die erfindungsgemäßen Zubereitungen auch Repellentien zum Schutz vor Mücken, Zecken und Spinnen und dergleichen enthalten. Vorteilhaft sind z. B. N,N-Diethyl-3-methylbenzamid (Handelsbezeichnung: Meta-delphene, "DEET"), Dimethylphtalat (Handelsbezeichnung: Palatinol M, DMP), 1-Piperidincarbonsäure-2-(2-hydroxyethyl)-1-methylpropylester sowie insbesondere 3-(N-n-Butyl-N-acetyl-amino)-propionsäure-ethylester (unter dem Handelsnamen Insekt Repellent® 3535 bei der Fa. Merck erhältlich). Die Repellentien können sowohl einzeln als auch in Kombination eingesetzt werden.

Als Feuchthaltemittel (Moisturizer) werden Stoffe oder Stoffgemische bezeichnet, welche kosmetischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.

Vorteilhafte Feuchthaltemittel (Moisturizer) im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist. Moisturizer können vorteilhaft auch als Antifaltenwirkstoffe zum Schutz vor Hautveränderungen, wie sie z. B. bei der Hautalterung auftreten, verwendet werden.

Es ist vorteilhaft im Sinne der vorliegenden Erfindung, wenn die erfindungsgemäße Zubereitung ein oder mehrere Feuchthaltemittel in einer Gesamtkonzentration von 0,1 bis 20 Gewichts-% und bevorzugt in einer Gesamtkonzentration von 0,5 bis 10 Gewichts-%, jeweils bezogen auf das Gesamtgewicht der Zubereitung, enthält.

Die erfindungsgemäßen kosmetischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile® (CAS-Nr. 7631-86-9).

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische Anwendungen geeigneten oder gebräuchlichen Antioxidantien verwendet werden.

Besonders vorteilhaft im Sinne der vorliegenden Erfindung können wasserlösliche Antioxidantien eingesetzt werden, wie beispielsweise Vitamine, z. B. Ascorbinsäure und deren Derivate.

Bevorzugte Antioxidantien sind ferner Vitamin E und dessen Derivate sowie Vitamin A und dessen Derivate.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Es ist insbesondere vorteilhaft, wenn die kosmetischen Zubereitungen gemäß der vorliegenden Erfindung kosmetische Wirkstoffe enthalten, wobei bevorzugte Wirkstoffe Antioxidantien sind, welche die Haut vor oxidativer Beanspruchung schützen können.

Weitere vorteilhafte Wirkstoffe im Sinne der vorliegenden Erfindung sind natürliche Wirkstoffe und/oder deren Derivate, wie z. B. alpha-Liponsäure, Phytoen, D-Biotin, Coenzym Q10, alpha-Glucosylrutin, Carnitin, Carnosin, natürliche und/oder synthetische Isoflavonoide, Kreatin, Kreatinin, Taurin und/oder β-Alanin sowie 8-Hexadecen-1,16-dicarbonsäure (Dioic acid, CAS-Nummer 20701-68-2; vorläufige INCI-Bezeichnung Octadecendioic acid) und/oder Licochalcon A, welches sich durch die folgende Strukturformel auszeichnet:

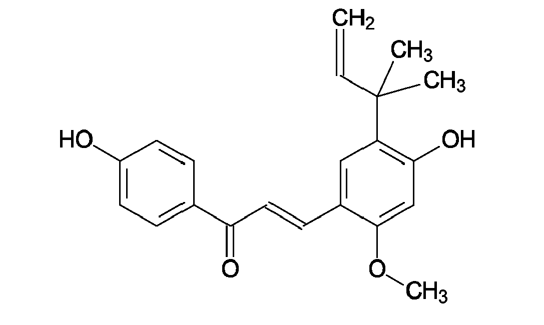

Licochalcon kann vorteilhaft auch als Bestandteil von pflanzlichen Extrakten, insbesondere von wäßrigen *Radix Glycyrrhizae inflatae*, eingesetzt werden.

Es ist erfindungsgemäß vorteilhaft, wenn die kosmetischen Zubereitungen 0,001 bis 10 Gew.-%, insbesondere 0,05 bis 5 Gew.-%, ganz besonders 0,01 bis 2 Gew.-% an einem Extrakt aus *Radix Glycyrrhizae inflatae* enthalten, jeweils bezogen auf das Gesamtgewicht der Zubereitung.

Ganz besonders vorteilhaft ist es, von einem Extrakt auszugehen, der unter der Bezeichnung Polyol Soluble Licorice Extract PU (INCI-Bezeichnung Glycyrrhiza Inflata) von der Firma Maruzen zu erhalten ist. Der Extrakt aus *Radix Glycyrrhizae inflatae* enthält einen Anteil von ca. 25 % Licochalcone A.

Erfindungsgemäße Rezepturen, welche z. B. bekannte Antifaltenwirkstoffe wie Flavonglycoside (insbesondere α-Glycosylrutin), Coenzym Q10, Vitamin E und/oder Derivate und dergleichen enthalten, eignen sich insbesondere vorteilhaft zum Schutz vor ästhetisch unattraktiven Hautveränderungen, wie sie z. B. bei der Hautalterung auftreten (wie beispielsweise Trockenheit, Rauhigkeit und Ausbildung von Trockenheitsfältchen, Juckreiz, verminderte Rückfettung (z. B. nach dem Waschen), sichtbare Gefäßerweiterungen (Teleangiektasien, Cuperosis), Schlaffheit und Ausbildung von Falten und Fältchen, lokale Hyper-, Hypo- und Fehlpigmentierungen (z. B. Altersflecken), vergrößerte Anfälligkeit gegenüber mechanischem Stress (z. B. Rissigkeit) und dergleichen). Weiterhin vorteilhaft eignen sie sich gegen das Erscheinungsbild der trockenen bzw. rauhen Haut.

Die kosmetischen Zubereitungen gemäß der Erfindung können kosmetische Hilfsstoffe enthalten, wie sie üblicherweise in solchen Zubereitungen verwendet werden, z. B. Konservierungsmittel, Konservierungshelfer, Komplexbildner, Bakterizide, Parfüme, Substanzen zum Verhindern oder Steigern des Schäumens, Farbstoffe, Pigmente, die eine färbende Wirkung haben, Verdickungsmittel, anfeuchtende und/oder feuchhaltende Substanzen, Füllstoffe, die das Hautgefühl verbessern, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Vorteilhaft im Sinne der vorliegenden Erfindung sind Zubereitungen zur Pflege der Haut: sie können dem kosmetischen Lichtschutz, ferner als Schminkprodukt in der dekorativen Kosmetik dienen.

Entsprechend ihrem Aufbau können kosmetische Zusammensetzungen im Sinne der vorliegenden Erfindung, beispielsweise verwendet werden als Hautschutzcreme, Tages- oder Nachtcrème usw. Es ist gegebenenfalls möglich und vorteilhaft, die erfindungsgemäßen Zusammensetzungen als Grundlage für pharmazeutische Formulierungen zu verwenden.

Es ist auch vorteilhaft im Sinne der vorliegenden Erfindung, kosmetische Zubereitungen zu erstellen, deren hauptsächlicher Zweck nicht der Schutz vor Sonnenlicht ist, die aber dennoch einen Gehalt an UV-Schutzsubstanzen enthalten. So werden z. B. in Tagescrèmes oder Makeup-Produkten gewöhnlich UV-A- bzw. UV-B-Filtersubstanzen eingearbeitet. Auch stellen UV-Schutzsubstanzen, ebenso wie Antioxidantien und, gewünschtenfalls, Konservierungsstoffe, einen wirksamen Schutz der Zubereitungen selbst gegen Verderb dar. Günstig sind ferner kosmetische Zubereitungen, die in der Form eines Sonnenschutzmittels vorliegen.

Zur Anwendung werden die erfindungsgemäßen kosmetischen Zubereitungen in der für Kosmetika üblichen Weise auf die Haut und/oder die Haare in ausreichender Menge aufgebracht.

### Vergleichsversuche

### 1. Stabilität von Mischungen aus 4,4',4"-(1,3,5-Triazin-2,4,6-triyltriimino)-tris-benzoësäure-tris(2-ethylhexylester) und 2,4-bis-[5-1(dimethylpropyl)benzoxazol-2-yl-(4-phenyl)-imino]-6-(2-ethylhexyl)-imino-1,3,5-triazin

Es wurden jeweils 2 Gewichts-% der UV-Filter isoliert in der angegebenen Ölkomponenten gelöst, die Ölphasen unter Rühren bei Raumtemperatur vereinigt und anschließend bei Raumtemperatur gelagert. Anschließend wurde die Mischung in den angegebenen Zeitabständen optisch auf Ausfällungen bzw. Transparenz hin untersucht.

| **Ölkomponente** | **24 Std.** | **2 Tage** | **4 Wochen** |
|---|---|---|---|
| Ricinusöl | transparent | transparent | Ausfällung/Trübung |
| Cocoglycerid | transparent | Ausfällung/Trübung | Ausfällung/Trübung |
| Di-C12-13 Alkyltartrat | transparent | transparent | transparent |
| Diethylhexyl 2,6-Naphthalat | Ausfällung/Trübung | Ausfällung/Trübung | Ausfällung/Trübung |
| Dibutyladipat | Ausfällung/Trübung | Ausfällung/Trübung | Ausfällung/Trübung |
| Butylenglycol Dicaprylat/Dicaprat | Ausfällung/Trübung | Ausfällung/Trübung | Ausfällung/Trübung |
| C12-15 Alkylbenzoat | Ausfällung/Trübung | Ausfällung/Trübung | Ausfällung/Trübung |
| Dicaprylylcarbonat | Ausfällung/Trübung | Ausfällung/Trübung | Ausfällung/Trübung |
| Caprylic/Capric Triglycerid | Ausfällung/Trübung | Ausfällung/Trübung | Ausfällung/Trübung |
| Octyldodecanol | Ausfällung/Trübung | Ausfällung/Trübung | Ausfällung/Trübung |
| Glyceryl Triheptanoat | transparent | Ausfällung/Trübung | Ausfällung/Trübung |

Wie sich aus dem beispielhaften Vergleichsversuch ergibt, sind die erfindungsgemäßen Lösungen mit Di-C12-13 Alkyltartrat besonders stabil.

### 2. Konstanz der Viskosität erfindungsgemäßer Zubereitungen

In drei weiteren Vergleichsversuchen wurde eine kosmetische Emulsionen enthaltend die erfindungsgemäße Kombination aus 4,4',4"-(1,3,5-Triazin-2,4,6-triyltriimino)-tris-benzoësäure-tris(2-ethylhexylester) (= Ethylhexyl Triazon) und 2,4-bis-[5-1 (dimethyl-propyl)benzoxazol-2-yl-(4-phenyl)-imino]-6-(2-ethylhexyl)-imino-1,3,5-triazin (=UVASORB K2A) mit und ohne Di-C12-13 Alkyltartrat hergestellt und die Viskosität nach einem bzw. 14 Tagen gemessen.

### O/W-Lotion mit niedrigem Lichtfiltergehalt

| | O/W-Lotion mit niedrigem Lichtfiltergehalt | + 5% Di-C12-13 Alkyltartrat |
|---|---|---|
| Cetylalkohol | 1,5 | 1,5 |
| Hydriertes Coco-Glycerid | 1 | 1 |
| C12-15 Alkylbenzoat | 10 | 7,5 |
| Ethylhexyl Triazon | 2 | 2 |
| Glycerylstearatcitrat | 2 | 2 |
| Di-C12-13 Alkyltartrat | 0 | 5 |
| Butylenglycol Dicaprylat/Dicaprat | 10 | 7,5 |
| UVASORB K2A | 2 | 2 |
| Glycerin | 5 | 5 |
| Trisodium EDTA | 1 | 1 |
| Xanthangummi | 0,3 | 0,3 |
| Wasser | ad 100 | ad100 |

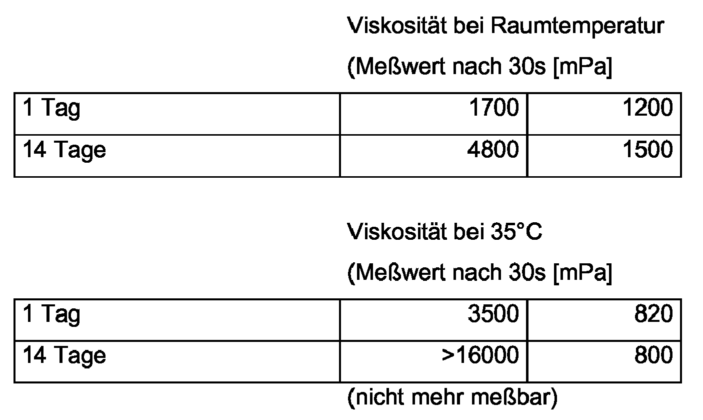

### O/W-Lotion mit hohem Lichtfiltergehalt

| | O/W-Lotion mit hohem Lichtfiltergehalt | + 5% Di-C12-13 Alkyltartrat |
|---|---|---|
| Cetyl Alcohol | 1,5 | 1,5 |
| Glyceryl Stearate SE | 1 | 1 |
| Ethylhexyl Methoxycinnamat | 7,5 | 7,5 |
| Cyclomethicon | 2 | 2 |
| Cetearyl Alcohol + PEG-40 Castor Oil + Sodium Cetearyl Sulfat | 2,5 | 2,5 |
| Hydriertes Coco-Glycerid | 0,5 | 0,5 |
| VP/Hexadecene Copolymer | 0,5 | 0,5 |
| Ethylhexyl Triazon | 2 | 2 |
| Titandioxid | 5 | 5 |
| C18-36 Acid Triglycerid | 1 | 1 |
| Bis-Ethylhexyloxyphenol Methoxyphenyl Triazin | 3 | 3 |
| Di-C12-13 Alkyl Tartrat | 0 | 5 |
| Butylen Glycol Dicaprylate/Dicaprat | 9,5 | 4,5 |
| UVASORB K2A | 2 | 2 |
| Tocopheryl Acetate | 0,5 | 0,5 |
| Phenylbenzimidazole Sulfonsäure | 2 | 2 |
| Xanthan Gum | 0,4 | 0,4 |
| Glycerin | 7,5 | 7,5 |
| Trisodium EDTA | 1 | 1 |
| Wasser | ad 100 | ad 100 |

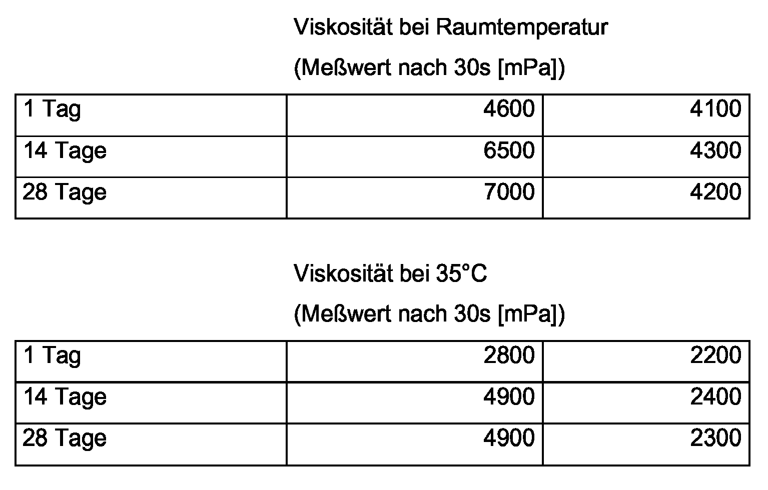

### W/O-Lotion mit hohem Lichtfiltergehalt

| | W/O-Lotion mit hohem Lichtfiltergehalt | + 5% Di-C12-13 Alkyltartrat |
|---|---|---|
| Ethylhexyl Methoxycinnamat | 7,5 | 7,5 |
| C12-15 Alkyl Benzoat | 6,5 | 6,25 |
| Butyl Methoxydibenzoylmethan | 4,5 | 4,5 |
| Isopropyl Stearat | 2,5 | 2,5 |
| Ethylhexyl Triazon | 2 | 2 |
| Titandioxid | 5 | 5 |
| C18-36 Acid Triglycerid | 0,5 | 0,5 |
| Ethylhexylglycerin | 0,5 | 0,5 |
| Polyglyceryl-2 Dipolyhydroxystearat | 6 | 6 |
| Octocrylen | 3,5 | 3,5 |
| Sodium Starch Octenylsuccinat | 0,5 | 0,5 |
| Bis-Ethylhexyloxyphenol Methoxyphenyl Triazin | 2 | 2 |
| Cetyl Dimethicon | 0,5 | 0,5 |
| Di-C12-13 Alkyl Tartrat | 0 | 5 |
| Butylen Glycol Dicaprylat/Dicaprat | 6,5 | 1,75 |
| UVASORB K2A | 2 | 2 |
| Tocopheryl Acetate | 0,5 | 0,5 |
| Magnesium Sulfate | 0,3 | 0,3 |
| Glycerin | 5 | 5 |
| Trisodium EDTA | 1 | 1 |
| Butylen Glycol | 5 | 5 |
| Alkohol | 2 | 2 |
| Glycin | 0,5 | 0,5 |
| Wasser | ad 100 | ad 100 |

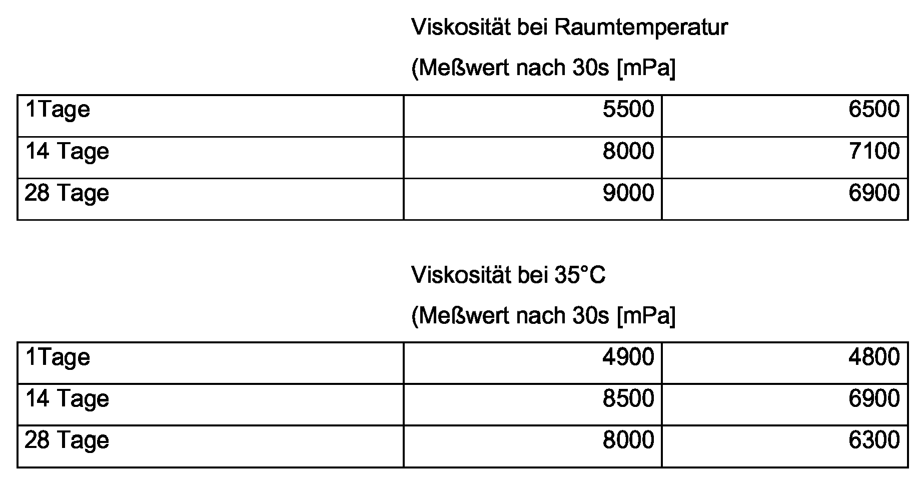

Wie sich aus dem beispielhaften Vergleichsversuchen ergibt, sind die erfindungsgemäßen Emulsionen mit Di-C12-13 Alkyltartrat besonders stabil.

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Beispiele:

### 1. O/W Sonnenschutz Emulsionen

| | **1** | **2** | **3** | **4** | **5** | **6** | **7** | **8** | **9** |
|---|---|---|---|---|---|---|---|---|---|
| Glycerinmonostearat SE | | 1,00 | 0,50 | 1,00 | 3,00 | | | 1,50 | |
| Glyceryl Stearat Citrat | | | 2,00 | | | 1,00 | 2,00 | | 2,50 |
| Stearinsäure | | | | 3,00 | 0,75 | 2,00 | | | |
| PEG-40 Stearat | | | 0,50 | | | | | 2,00 | |
| Cetearylalkohol + PEG-40 Ricinusöl + Sodium Cetearyl Sulfat | | 2,50 | | | | | | | |
| Polyglyceryl-3 Mehylglucose Distearat | 2,50 | | | | | | | | |
| Sorbitan Stearat | 1,00 | | | | | | | | |
| Cetyl Dimethicon Copolyol | | | | | | 0,75 | | 0,50 | |
| Cetyl Phosphat | | | | | 0,75 | | | | |
| Stearyl Alkohol | 0,50 | | | | 3,00 | | | 2,00 | 0,50 |
| Cetyl Alkohol | 0,50 | 1,50 | 2,50 | 1,00 | | | 1,50 | | 2,00 |
| UVASorb® K2A | 2,00 | 2,00 | 1,00 | 2,50 | 3,00 | 4,00 | 1,50 | 5,00 | 1,00 |
| Butyl Methoxydibenzoylmethan | 2,00 | | | | | | | | |
| Bis-Ethylhexyloxyphenol Methoxyphenyl Triazin | | 3,00 | | | 0,50 | | | 1,00 | |
| Phenylbenzmidazol Sulfonsäure | | | | 0,50 | | | | 2,00 | |
| Ethylhexyl Triazon | | 2,00 | 3,00 | 2,00 | 1,00 | 2,00 | 2,00 | 3,00 | |
| Diethylhexyl Butamido Triazon | 4,00 | | | 2,00 | | | | | 3,00 |
| Ethylhexyl Methoxycinnamat | | | | 3,50 | | 10,00 | | | |
| Octocrylen | | | | | | 5,00 | | 7,50 | 2,50 |
| Methylen Bis-Benztriazolyl Tetramethylbutylphenol | | | 2,00 | | 0,50 | | | | |
| Ethylhexylsalicylat | | | | | 3,00 | | | | 5,00 |
| Drometrizol Trisiloxan | | | | | 0,5 | | | 1,00 | |

| | | | | | | | | | |
|---|---|---|---|---|---|---|---|---|---|
| Titandioxid T 805 | | 5,00 | | 1,50 | | | | 0,50 | |
| Titandioxid MT-100Z | | | 1,00 | | | 3,00 | | | |
| Hydriertes Cocoglycerid | | 0,50 | | | | | 1,00 | | |
| C18-38Fessäuretriglycerid | | 1,00 | | | | | | | |
| C12-15 Alkyl Benzoat | | | | 2,50 | | | 7,50 | 7,00 | 5,00 |
| Dicaprylyl Ether | | | | | 3,50 | | | | |
| Butylenglycol Dicaprylat/Dicaprat | | 4,50 | 5,00 | | | 5,00 | 5,00 | | |
| Di C12-13 Alkyl Tartrat | 7,00 | 5,00 | 5,00 | 3,00 | 5,00 | 10,00 | 5,00 | 3,00 | 3,00 |
| Dicaprylylcarbonat | 2,00 | | | | | | | | |
| Cetearyl Isononanoate | | | | 4,00 | | | | 2,00 | 2,00 |
| Dimethicon | | | | 0,50 | 1,00 | | 2,00 | | |
| Cyclomethicon | | | 2,00 | | | 4,50 | | | 0,50 |
| Shea Butter | | | | 2,00 | | | | | 0,50 |
| PVP Hexadecen Copolymer | | 0,50 | 0,50 | | | 0,50 | | | 1,00 |
| Phenylbenzimidazolsulfon säure | | 2,00 | | | | | | | |
| Glycerin | 10,00 | 7,50 | 3,00 | 7,50 | | 7,50 | 5,00 | | 2,50 |
| Xanthan Gummi | 0,20 | 0,40 | 0,15 | | 0,05 | | 0,30 | | 0,30 |
| Acrylate/C 10-30 Alkyl Acrylat Corosspolymer | 0,10 | | | | | | | | |
| Natronlauge 45% | 0,10 | 0,70 | | | | | | | |
| Butylene Glycol | | | | 5,00 | | | | 7,00 | |
| Vitamin E Acetat | | 0,50 | 0,50 | | 0,25 | 0,50 | 0,75 | | 1,00 |
| Alpha-Glucosylrutin | | | 0,25 | | | 0,20 | | 0,25 | |
| Fucogel® 1000 | | | | | 1,50 | | | 5,00 | |
| DMDM Hydantoin | | | | 0,60 | 0,40 | 0,20 | | | |
| Methylparaben | 0,10 | | 0,15 | | 0,25 | | 0,50 | | |
| Ethylparaben | 0,10 | 0,20 | | | | | | | |
| Ethylhexylglycerin | 0,50 | | | | | | | | |
| Phenoxyethanol | 0,50 | 0,30 | 1,00 | 0,40 | | 0,40 | 0,50 | | 0,60 |
| EDTA | | 1,00 | | 0,20 | 0,35 | 0,50 | 0,02 | | 0,03 |

| | | | | | | | | | |
|---|---|---|---|---|---|---|---|---|---|
| Ethanol | | | | 2,00 | 1,50 | | 3,00 | 5,00 | 1,00 |
| Insekt Repellent 3535 | | | | | | | | 10,00 | |
| Parfüm | | | 0,20 | 0,20 | | | | 0,30 | 0,40 |
| Wasser | ad 100 | ad 100 | ad 100 | ad 100 | ad 100 | ad 100 | ad 100 | ad 100 | ad 100 |

### 2. schaumförmige O/W- Emulsionen:

| | Emulsion 1 | | Emulsion 2 | |
|---|---|---|---|---|
| | GEW.-% | Vol.-% | GEW.-% | Vol.-% |
| Stearinsäure | 5,00 | | 1,00 | |
| Cetylalkohol | 5,50 | | | |
| Cetylstearylalkohol | | | 2,00 | |
| PEG-40 Stearat | 8,50 | | | |
| PEG-20-Stearat | | | 1,00 | |
| Caprylsäure/Caprinsäuretriglyceride | 4,00 | | 2,00 | |
| C12-15 Alkyl Benzoat | 5,00 | | 10,00 | |
| Di- C12-13 Alkyltartrat | 5,00 | | 5,00 | |
| Cyclomethicon | 4,00 | | | |
| Dimethicon | | | 0,50 | |
| Octyl isostearat | | | 5,00 | |
| Myristyl Myristat | | | 2,00 | |
| Ceresin | 1,50 | | | |
| Glycerin | | | 3,00 | |
| UVASorb® K2A | 2,00 | | 4,00 | |
| Bis-Ethylhexyloxyphenol Methoxyphenyl Triazin | 3,00 | | 2,50 | |
| Phenylbenzmidazol Sulfonsäure | | | 0,50 | |
| Disodium Phenyl Dibenzimidazol Tetrasulfonat | 2,00 | | 1,50 | |
| Terephthaliden Dicamphor Sulfonsäure | 0,50 | | | |
| Ethylhexylmethoxycinnamat | 5,00 | | 4,00 | |

| | | | | |
|---|---|---|---|---|
| Ethylhexyltriazon | 2,00 | | 3,00 | |
| Octocrylene | 5,00 | | | |
| Titandioxid MT-100 TV | 1,00 | | | |
| BHT | | | 0,02 | |
| Na₂H₂EDTA | 0,50 | | 0,10 | |
| Parfüm, Konservierungsmittel, | Q.S. | | Q.S. | |
| Farbstoffe, usw. | Q.S. | | Q.S. | |
| Kaliumhydroxid | Q.S | | Q.S | |
| Wasser | AD 100,00 | | AD 100,00 | |

| | | | | |
|---|---|---|---|---|
| | pH-Wert eingestellt auf 6,5-7,5 | | pH-Wert eingestellt auf 5,0-6,0 | |
| **Emulsion 1** | | 70 | | |
| **Emulsion 2** | | | | 35 |
| Gas (Stickstoff) | | 30 | | |
| Gas (Helium) | | | | 65 |

Vereinigung der auf 78 °C aufgeheizten Fett-/Lichtschutzfilterphase mit der auf 75 °C aufgeheizten Wasser-/Lichtschutzfilterphase. Homogenisierung mittels einer Zahnkranzdispergiermaschine (Rotor-Stator-Prinzip) bei 65 °C. 45 min Rühren im Becomix unter Begasung mit Helium bei 1 bar unter Kühlung auf 30 °C. Zugabe der Additive bei 30 °C (Parfüm). Homogenisierung mittels einer Zahnkranzdispergiermaschine (Rotor-Stator-Prinzip) bei 23 °C.

### 3. PIT-Emulsionen (zur Verwendung als Tränkungslösung, Spray oder Aerosol)

| | **1** | **2** | **3** | **4** | **5** | **6** | **7** | **8** |
|---|---|---|---|---|---|---|---|---|
| Glycerinmonostearat SE | 0,50 | 2,00 | 3,00 | 5,00 | | | 0,50 | 4,00 |
| Glyceryl Isostearat | | | | | 3,50 | 4,00 | 2,00 | |
| Isoceteth-20 | | 0,50 | | | 2,00 | | | |
| Ceteareth-12 | | 5,00 | | 1,00 | | | | 3,50 |
| Ceteareth-20 | | | | 2,00 | | 2,50 | 3,00 | |
| PEG-100 Stearat | 5,00 | | 1,00 | | | | | 0,50 |
| Cetyl Alkohol | 2,50 | 1,00 | | 1,50 | | 0,50 | 1,50 | |
| Cetyl Palmitat | | | | 0,50 | | 1,00 | | |
| Cetyl Dimethicon Copolyol | 0,50 | | | | 0,50 | | 1,00 | |

| | | | | | | | | |
|---|---|---|---|---|---|---|---|---|
| UVASorb® K2A | 1,50 | 2,00 | 2,00 | 3,00 | 5,00 | 3,00 | 1,00 | 3,50 |
| Bis-Ethylhexyloxyphenol Methoxyphenyl Triazin | | | | 0,75 | 0,25 | | 2,00 | |
| Dinatrium Phenyl Dibenzimidazol Tetrasulfonat | | 2,00 | | | 1,00 | | | 2,00 |
| Terephthaliden Dicampher Sulfonsäure | | | 0,50 | | | | 1,00 | |
| Butyl Methoxydibenzoylmethan | 1,50 | | 1,00 | | | | 0,75 | |
| Drometrizol Trisiloxan | | | 2,00 | | | 3,00 | | 1,00 |
| Ethylhexyl Methoxycinnamat | 8,00 | | | 4,50 | 5,00 | 5,00 | | |
| Diethylhexyl Butamido Triazon | 2,00 | 0,50 | | | 2,00 | 2,00 | | 1,50 |
| Ethylhexyl Triazon | | 0,50 | 2,00 | 4,00 | | 2,00 | 1,50 | 3,00 |
| Octocrylen | | | | | 3,00 | | | 7,50 |
| C12-15 Alkyl Benzoat | 3,50 | | | | | | | |
| Di-C12-13 Alkyltartrat | 5,00 | 4,00 | 3,00 | 5,00 | 7,00 | 5,00 | 2,00 | 5,00 |
| Cocoglyceride | | 3,00 | | 3,00 | | | | 3,50 |
| Butylenglycol Dicaprylat/Dicaprat | | 2,00 | | | | 3,00 | | |
| Dicaprylyl Carbonat | | | 2,00 | | | | | 6,00 |
| Phenyltrimethicon | 2,00 | | | | 2,00 | | | |
| PVP Hexadecen Copolymer | | | | 1,00 | 1,50 | | | |
| Glycerin | 10,0 | 5,00 | | 7,50 | | 10,00 | | |
| Fucogel®1000 | | | 2,50 | 6,00 | | | | |
| Tocopherol | 1,00 | | | 0,75 | 0,50 | | 1,00 | |
| Sonnenblumenöl | | 2,00 | 3,50 | | | | | 0,50 |
| lodopropyl Butylcarbamat | 0,12 | | | | 0,20 | | | |
| DMDM Hydantoin | | | | 0,10 | | | | |
| Methylparaben | | 0,50 | 0,25 | | 0,45 | | | |
| Phenoxyethanol | 0,50 | 0,40 | | 1,00 | | | | 1,00 |
| Ethylhexyloxyglycerin | | 0,30 | | | 1,00 | 0,35 | | |
| Ethanol | | | | 2,00 | | 6,00 | 7,50 | 4,00 |
| Trisodium EDTA | | 0,40 | | 0,15 | | 0,20 | | 0,50 |
| Parfüm | 0,20 | | 0,20 | 0,20 | 0,45 | | | 0,20 |
| Wasser | ad. | ad. | ad. | ad. | ad. | ad. | ad. | ad. |

| | | | | | | | | |
|---|---|---|---|---|---|---|---|---|
| | 100 | 100 | 100 | 100 | 100 | 100 | 100 | 100 |

### 1. Dünnflüssige bis sprühbare W/O-Emulsionen (zur Verwendung als Tränkungslösung, Spray oder Aerosol)

| | **1** | **2** | **3** | **4** | **5** |
|---|---|---|---|---|---|
| Cetyl Dimethicon Copolyol | 4,00 | | | 2,50 | 3,00 |
| Polyglyceryl-2 Dipolyhydroxystearat | | | 3,00 | | 1,00 |
| Isostearyl Diglyceryl Succinat | | | 0,75 | | 0,30 |
| Lauryl Methicon Copolyol | | | | 2,00 | |
| Polysorbat-65 | | | 2,00 | | 1,50 |
| PEG-100 Stearat | | | | 1,20 | 0,70 |
| Cetearyl Sulfat | | | 0,25 | | 1,00 |
| Dimethicon | | 6,00 | | | 2,00 |
| Cyclomethicon | 12,00 | | | 30,00 | 15,00 |
| UVASorb® K2A | 2,00 | 1,50 | 3,00 | 0,50 | 5,00 |
| Butylmethoxy Dibenzoylmethan | 0,25 | | | 1,00 | |
| Phenylbenzmidazol Sulfonsäure | | 1,50 | 2,00 | | 2,00 |
| Drometrizol Trisiloxan | | | | 1,00 | |
| Ethylhexyl Methoxycinnamat | 3,00 | 4,00 | | | 10,00 |
| Octocrylen | | 5,00 | | 4,00 | 7,50 |
| Diethylhexyl Butamido Triazon | | 1,00 | | | 6,50 |
| Ethylhexyl Triazon | 3,00 | | 2,00 | 3,00 | 4,00 |
| Titandioxid MT-100 TV | | 0,50 | 1,00 | 1,50 | 0,50 |
| Zinkoxid Z-Cote HP1 | 2,00 | | | | 4,00 |
| Dicaprylyl Carbonat | 5,00 | | 10,00 | | |
| Dihexyl Carbonat | | 10,00 | | | |
| C12-15 Alkyl Benzoat | 7,00 | | 10,00 | | |
| Di-C12-13 Alkyltartrat | 10,00 | 8,00 | 5,00 | 5,00 | 8,00 |
| Mineral Öl | 3,00 | | | | 3,00 |
| 2,6 Diethylhexylnaphthalat | | 2,00 | | | |
| PVP Hexadecen Copolymer | | 0,75 | | | 0,40 |
| Glycerin | | | | 5,00 | 7,00 |
| α-Glucosylrutin | | | | | 0,15 |
| EDTA | | 0,15 | 0,03 | | 0,15 |

| | | | | | |
|---|---|---|---|---|---|
| Glycin Soja | 0,75 | | | 1,50 | |
| Magnesiumsulfat | 0,75 | 1,00 | | 0,45 | 1,00 |
| DMDM Hydantoin | | 0,05 | | | 0,10 |
| Phenoxyethanol | 1,00 | 0,75 | 0,50 | | 1,00 |
| Ethanol | 2,00 | | | 5,00 | 1,00 |
| Farbstoff | 0,02 | | | | |
| Parfüm | 0,30 | 0,45 | 0,35 | | 0,15 |
| Wassser | ad. 100 | ad. 100 | ad. 100 | ad. 100 | ad. 100 |

## Patentansprüche

1. Kosmetische Zubereitung enthaltend
a) eine UV-Lichtschutzfilterkombination aus
i) 4,4',4"-(1,3,5-Triazin-2,4,6-triyltriimino)-tris-benzoësäure-tris(2-ethylhexylester) und/oder Dioctylbutylamidotriazon sowie
ii) 2,4-bis-[5-1(dimethylpropyl)benzoxazol-2-yl-(4-phenyl)-imino]-6-(2-ethylhexyl)-imino-1,3,5-triazin und
b) eine oder mehrere Ölkomponenten mit mindestens einer Hydroxyl-Gruppe, die **dadurch gekennzeichnet ist, dass** der oder die Ölkomponenten mit mindestens einer Hydroxyl-Gruppe zu mindestens 10 Gewichts-%, bezogen auf das Gesamtgewicht der Ölphase, in der Zubereitung enthalten sind.

2. Verwendung von Ölkomponenten mit mindestens einer Hydroxyl-Gruppe zur Reduzierung der Viskosität von kosmetischen Zubereitungen welche eine UV-Lichtschutzfilterkombination aus
i) 4,4',4"-(1,3,5-Triazin-2,4,6-triyltriimino)-tris-benzoësäure-tris(2-ethylhexylester) und/oder Dioctylbutylamidotriazon sowie
ii) 2,4-bis-[5-1(dimethylpropyl)benzoxazol-2-yl-(4-phenyl)-imino]-6-(2-ethylhexyl)-imino-1,3,5-triazin, enthalten.

3. Kosmetische Zubereitung oder Verwendung nach einem der vorhergehenden Ansprüche, **dadurch gekennzeichnet, dass** als Ölkomponente ein Dihydroxydicarbonsäureester eingesetzt wird.

4. Kosmetische Zubereitung oder Verwendung nach einem der vorhergehenden Ansprüche, **dadurch gekennzeichnet, dass** als Ölkomponente das Di-C12-13 Alkyltartrat gewählt wird.

5. Kosmetische Zubereitung oder Verwendung nach einem der vorhergehenden Ansprüche, **dadurch gekennzeichnet, dass** eine UV-Lichtschutzfilterkombination aus 4,4',4"-(1,3,5-Triazin-2,4,6-triyltriimino)-tris-benzoësäure-tris(2-ethylhexylester) und 2,4-bis-[5-1 (dimethylpropyl)benzoxazol-2-yl-(4-phenyl)-imino]-6-(2-ethylhexyl)-imino-1,3,5-triazin eingesetzt wird.

6. Kosmetische Zubereitung oder Verwendung nach einem der vorhergehenden Ansprüche, **dadurch gekennzeichnet, dass** die UV-Lichtschutzfilterkombination in einer Gesamtmenge von 0,5 bis 8 Gewichts-%, bezogen auf das Gesamtgewicht der Zubereitung, in der Zubereitung enthalten ist.

7. Kosmetische Zubereitung oder Verwendung nach einem der vorhergehenden Ansprüche, **dadurch gekennzeichnet, dass** das Gewichtsverhältnis von 4,4',4"-(1,3,5-Triazin-2,4,6-triyltriimino)-tris-benzoësäure-tris(2-ethylhexylester) zu 2,4-bis-[5-1(dimethylpropyl)benzoxazol-2-yl-(4-phenyl)-im ino]-6-(2-ethyl hexyl)-imino-1,3,5-triazi n von 0,5 : 5 bis 5 : 0,5 beträgt.

8. Kosmetische Zubereitung oder Verwendung nach einem der vorhergehenden Ansprüche, **dadurch gekennzeichnet, dass** das Gewichtsverhältnis von UV-Lichtschutzfilterkombination zur Gesamtmenge an Ölkomponenten mit mindestens einer Hydroxyl-Gruppe von 1,5 : 1 bis 1 : 10 beträgt.

9. Kosmetische Zubereitung oder Verwendung nach einem der vorhergehenden Ansprüche, **dadurch gekennzeichnet, dass** die Viskosität der Zubereitung weniger als 15000 mPas beträgt.

10. Kosmetische Zubereitung oder Verwendung nach einem der vorhergehenden Ansprüche, **dadurch gekennzeichnet, dass** die Zubereitung in Form einer Emulsion vorliegt.
